(19) Europäisches Patentamt
European Patent Office
Office européen des brevets

(11) **EP 2 467 722 B1**

(12) **EUROPEAN PATENT SPECIFICATION**

(45) Date of publication and mention
of the grant of the patent:
**27.11.2013 Bulletin 2013/48**

(21) Application number: **10752428.2**

(22) Date of filing: **16.08.2010**

(51) Int Cl.:
***G01N 33/543*** *(2006.01)*

(86) International application number:
**PCT/IB2010/053690**

(87) International publication number:
**WO 2011/021142 (24.02.2011 Gazette 2011/08)**

(54) **DETECTION OF DIFFERENT TARGET COMPONENTS BY CLUSTER FORMATION**

NACHWEIS VERSCHIEDENER ZIELKOMPONENTEN DURCH AGGREGAT-BILDUNG

DÉTECTION DE COMPOSANTS CIBLES DIFFÉRENTS PAR LA FORMATION D' AGRÉGATS

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **19.08.2009 EP 09168150**

(43) Date of publication of application:
**27.06.2012 Bulletin 2012/26**

(73) Proprietor: **Koninklijke Philips N.V.
5656 AE Eindhoven (NL)**

(72) Inventors:
• **RANZONI, Andrea
NL-5656 AE Eindhoven (NL)**
• **OVSYANKO, Mikhail, Mikhaylovich
NL-5656 AE Eindhoven (NL)**
• **PRINS, Menno, Willem, Jose
NL-5656 AE Eindhoven (NL)**

(74) Representative: **van Velzen, Maaike Mathilde
Philips
Intellectual Property & Standards
P.O. Box 220
5600 AE Eindhoven (NL)**

(56) References cited:
EP-A1- 1 662 256      EP-A1- 1 992 938
WO-A1-2005/010527    WO-A1-2008/075285
WO-A1-2009/037636    WO-A1-2009/091643
US-A- 5 932 097      US-A1- 2006 040 286

**Description**

FIELD OF THE INVENTION

The invention relates to a method, an apparatus, and a test kit for detecting a plurality of different species of target components in a sample.

BACKGROUND OF THE INVENTION

The US 2008/0206104 A1 discloses a magnetic biosensor in which different target substances labeled with magnetic beads can specifically bind to antibodies on a sensor surface. To distinguish between different types of bindings, a rotating magnetic field is applied that tests the rotational and/or translational mobility of the magnetic beads.

The US 2006/040286 A1 discloses a procedure in which target molecules are bound between capture probes comprising a magnetic particle and detection probes comprising a DNA barcode. The resulting complexes are separated from the sample by magnetic forces, and the DNA barcodes in the separated fraction are detected.

According to the US 5 932 097 A, specific magnetic particles with different magnetic characteristics are used as labels for target molecules, wherein the magnetically labeled particles are spatially separated based on their different migration properties in an external magnetic field.

The WO 2009/091643 A1 discloses a method in which different cell populations are detected by binding to a first capture complex and a second capture complex, respectively. The capture complexes comprise magnetic particles and specific fluorescent labels. Cells bound to these complexes can magnetically be separated, wherein different cell populations can then be discriminated due to the specific fluorescent labels.

The WO 2009/037636 A1 discloses that magnetically labeled target components are detected with the help of a rotating magnetic field that has a frequency above the critical slipping frequency of the magnetic particle. The detection of different target species (multiplexing) is achieved with the help of magnetic particles that comprise different optical properties, e.g. different colors.

SUMMARY OF THE INVENTION

Based on this situation it was an object of the present invention to provide alternative means for the simultaneous detection of different species of target components in a sample, wherein it is desirable that the detection procedure is easy, fast and accurate.

This object is achieved by a method according to claim 1 and an apparatus according to claim 2. Preferred embodiments are disclosed in the dependent claims.

According to a first aspect, the invention relates to a method for detecting a (natural) number of $N \geq 2$ different species of target components in a sample. The "target components" may for example be biological substances like biomolecules, complexes, cell fractions or cells. The "sample" may for example be a fluid of biological origin like blood, urine, or saliva. The method comprises the following steps, which are preferably executed at least once in the listed sequence:

a) The addition of particles to the sample which shall be tested for the presence of target components, wherein these particles will be called "label particles" in the following. The added label particles can be subdivided into N classes such that label particles from each class can bind to each other via the same, class-specific species of target component. Moreover, the label particles of each class preferably have at least one (e.g. magnetic or mechanical) class-specific property in common. The expression "class-specific" denotes in this context that the respective property is (at least approximately) the same for any two label particles of one class, but differs from class to class such that any two label particles chosen from different classes differ in their properties. Similarly, any two label particles from the same class can bind to each other via the same species of target component, wherein this species of target component is however different from class to class. As an additional requirement, at least one label particle of each class shall be a magnetic particle (i.e. a magnetized or magnetizable particle). These magnetic particles are optionally superparamagnetic beads with a diameter in the range between 10 nm and 10 $\mu$m, preferably between 100 nm and 3 $\mu$m.

b) The step of allowing the label particles to form clusters. As usual, the term "cluster" shall denote an agglomerate of at least two label particles that are more or less strongly bound to each other. The formation of clusters may occur spontaneously (within a delay time) and/or it may be assisted, for example by the application of a magnetic field in the sample.

c) The selective actuation of different types of the aforementioned clusters, the actuation comprising an oscillating motion or a fully rotating motion of the clusters. The angular velocity of the full rotation can be uniform as well as varying over time (periodically and/or aperiodically). The "selective actuation" of different types of clusters shall mean that the magnetic field can deliberately be changed between (at least) a first and a second mode, wherein at least two of the considered cluster types change their reactions to these modes in a different way, which allows to distinguish them. In particular, the reactions of interest may be of an "all-or-nothing" manner such that one cluster type is actuated by the first mode only and not by the second mode, while another cluster type is actuated by both modes (or no mode).

d) The detection of the aforementioned selectively actuated clusters. By selecting in advance which types of clusters are actuated, this detection step allows to distinguish between the different types of clusters.

In step c), the clusters are excited in a rotational fashion (partial or complete rotation) by application of a time-varying magnetic field. The applied field generates a mechanical torque. The torque is caused by a magnetic property of the cluster, e.g. a permanent or an induced magnetic moment, a shape anisotropy, a magnetocrystalline anisotropy, or a finite magnetic relaxation time of the magnetic material.

According to a second aspect, the invention relates to an apparatus for detecting a number of $N \geq 2$ different species of target components in a sample, preferably with a method of the kind described above, said apparatus comprising the following components:

a) A sample chamber for accommodating the sample. The "sample chamber" typically comprises an empty cavity or a cavity filled with some substance like a gel that may absorb a sample substance; it may be an open cavity, a closed cavity, or a cavity connected to other cavities by fluid connection channels.

b) A magnetic field generator for applying a time-varying magnetic field to the sample chamber, wherein the magnetic field is adapted to selectively actuate different types of clusters (C 11, C22, C 12, C 11', C22', C 12', ... CNN) to perform an oscillating motion or a fully rotating motion, wherein the clusters comprise at least one class of label particles (1, 2, ... N), wherein at least one of the label particles (1, 2, ...N) in each class is a magnetic particle, and wherein label particles from each class can bind to label particles from the same class via a class-specific species of target component (T 1, T2, .... TN).

c) A detection system for detecting the aforementioned selectively actuated clusters of label particles.

The method and the corresponding apparatus described above allow the use of label particles that form clusters by binding to each other via specific target components, wherein said clusters may differ in their magnetic or mechanical properties if they comprise different target components (if binding specifity is paired with specific magnetic/mechanical properties). The different clusters can selectively be actuated and, accordingly, selectively be detected. As a result, it is possible to qualitatively and/or quantitatively determine the presence of different species of target components in a sample. As the required processing steps can be executed in the bulk medium of the sample, the corresponding assay is easy to execute, fast, and accurate.

In the following, various preferred embodiments of the invention will be described that relate to both the method and the apparatus described above.

Oscillation of clusters comprising at least one magnetic particle can be induced with a wide variety of magnetic field configurations. For example, creating a planar magnetic field characterized by the following two orthogonal components succeeds in inducing an oscillatory motion of two-particle clusters:

A first component can be used to induce a preferred orientation of the clusters. This component can be a constant field, a series of pulses of arbitrary length or a sinusoidal wave. The amplitude of this component should be tuned so that only clusters up to the size of interest can respond to the external actuation.

A second component is periodically turned on to induced magnetic repulsion between the label particles. The field configuration could be a series of pulses or a sinusoidal wave. The amplitude of this component should be at maximum 10 times bigger than the first component.

A description for a fully rotating field, in which amplitude, phase and frequency can have a time dependence, is comprised by formula (1).

$$\underline{B} = \begin{pmatrix} a \cdot \sin(2\pi f_1 t + \varphi) \\ b \cdot \cos(2\pi f_2 t + \phi) \end{pmatrix} \qquad (1)$$

wherein a, b are real numbers and $f_1$ and $f_2$ are the rotation frequencies of the two components. In a particular example, such a magnetic field may consist of two continuous sinusoidal components, wherein the two components differ in amplitude and/or phase. In a preferred embodiment, a magnetic field is used that has at least one oscillating component, wherein the field amplitude varies over time. In this context, a component of a field is called "oscillating" if it repetitively (periodically or aperiodically) increases and decreases in magnitude.

According to one embodiment, only clusters up to a predetermined size are actuated by the magnetic field, wherein the "size" of a cluster may for example relate to the weight of the cluster and/or the number of label particles in the cluster. In this way the analysis can be restricted to smaller clusters which show less variations in their possible configurations and properties. Moreover, the limitation of the effects of a magnetic field to smaller cluster sizes can readily be achieved by limiting the field amplitude because the field amplitude needed for actuating a cluster typically increases with increasing cluster size.

In the aforementioned embodiment, it is most preferred that only (at most) clusters consisting of two label particles are actuated by the magnetic field. This restricts the measurements to a limited number of different types of clusters, hence allowing a unique interpretation of measurement results. In particular, it is possible to distinguish two-particle clusters in which the label particles are specifically bound via different target components, because the bound particle pairs belong to different classes and accordingly may have different magnetic or mechanical properties. The considered clusters can hence be distinguished via their reaction to the actuating magnetic field.

The ratio between the maximum and minimum amplitudes of the magnetic field may typically range between 1.1 and 10, preferably between 2 and 8, and most preferably between 4 and 6. For these values, a favorable differentiation between different clusters has been observed.

The selective actuation of clusters that is achieved by the magnetic field with at least one oscillating component and varying field amplitude may preferably comprise the selective oscillation and/or (at least partial) rotation of said clusters. These are reactions of clusters to an external oscillating or rotating magnetic field that can usually always be provoked under appropriate operating conditions and that is stable and readily detectable.

In the aforementioned case, the detection of selectively actuated clusters preferably comprises the detection of an oscillation and/or a rotation of said clusters synchronously to the oscillating component of the magnetic field. Synchronicity of oscillation/rotation with the applied oscillating magnetic field is a behavior that can usually be observed for clusters under appropriate operating parameters, particularly for a rotating magnetic field. Moreover, said synchronicity often disappears at critical operating parameters which depend on the magnetic and/or mechanical properties of the cluster. Determination of these critical parameters hence provides information about the type of cluster at hand.

It was already mentioned that the magnetic field may particularly be a rotating magnetic field, i.e. a field with a (uniformly or non-uniformly) rotating magnetic field vector. The rotational frequency of such a rotating magnetic field is preferably swept over a given range. The (momentary) rotational frequency of the rotating magnetic field is defined in this context (up to a constant factor) by the angular velocity with which the field vector rotates at the considered moment in time. The rotational frequency of a rotating magnetic field is an important characteristic parameter of this field which determines if and how a cluster reacts. The rotational frequency can hence often be used to implement the cluster-selectivity of an actuation process. Sweeping this rotational frequency over a given range means that all values of this range are assumed at least once, typically in an ordered sequence of increasing or decreasing magnitude. It should be noted that the considered given range can be any set of frequency values, though continuous intervals described by a lower and an upper boundary are preferred.

In a concrete example of the aforementioned embodiment, the range through which the rotational frequency is swept comprises at least one frequency above and one frequency below a critical frequency, wherein said critical frequency is defined by the fact that one type of cluster changes (e.g. stops) its reaction to the rotating magnetic field at the critical frequency. If "actuation of the clusters" means for example that they rotate synchronously to the rotating magnetic field (i.e. with the momentary rotational frequency of this field), such a cluster may stop (or start) synchronous rotation when the rotational frequency passes the critical frequency. Detection of cluster rotation in combination with sweeping the rotational frequency of the magnetic field will hence allow to determine the critical frequency of a cluster, which in turn provides information about the label particles the cluster is composed of.

The detection of selectively actuated clusters can in principle be achieved with any method and device that is suited for this purpose. In a preferred embodiment, the selectively actuated clusters are optically detected with an optical detector. Optical detection has the advantage that it can be executed in the bulk without mechanical contact to a sample and without affecting processes therein. The optical detection may for example be based on light that is transmitted through a sample or reflected from a sample, wherein this transmission/reflection is characteristically affected by actuated clusters. For rotating clusters, scattering of transmitted/reflected light may for example readily be detected in the observed output light as an intensity variation synchronous to the rotation. Optical detection may also be based on detection by imaging, fluorescence, absorption, scattering, etc.

The actuation of the clusters of particles is preferably done in such a way that it breaks (only) a-specific clusters and hence improves the signal-to-noise ratio. Modulated magnetic fields may be used in this respect to induce oscillations/rotation of clusters implying repulsion between the particles stronger than a-specific interactions and weaker than the specific biological bond (cf. patent application EP08105253.2. ).

The magnetic field generator that is used to generate the magnetic field with varying field amplitude may particularly be realized by a multipole configuration of magnetic coils which are supplied with electrical currents according to an appropriate schedule.

In the following, various embodiments of the invention will be described that relate to the method and the apparatus according to the first and second aspect of the invention.

According to a preferred embodiment, all particles of at least one class are magnetic particles. Most preferably, the label particles of all classes are magnetic particles. This ensures that all particles in particle clusters are magnetic, which facilitates the magnetic actuation of the clusters.

The optional class-specific property of the label particles preferably comprises their magnetic susceptibility. The susceptibility determines the magnetic dipole moment a magnetic particle will assume in an external magnetic field and hence the force which can be exerted on said particle for actuation purposes. The susceptibility can for example be adjusted via the size of the magnetic particle, the relative amount of magnetically active material with respect to a magnetically inactive matrix material, the type of the magnetically active material or the like.

According to another embodiment, label particles from different classes have substantially (i.e. within limits of about ± 20 %, preferably ± 10 %) the same size. In this context, the term "size" may refer to the geometrical shape (volume), the hydrodynamic volume, and/or the weight of a particle. As the size of the label particles will usually have an influence on the way they react to an external magnetic field (e.g. due to effects of inertia or viscosity), the sizes of label particles from different classes are preferably similar or identical to each other to prevent that they affect the particle reaction. This embodiment is preferably combined with the aforementioned one of the differing magnetic susceptibilities, which guarantees that differentiating effects of susceptibility are not superimposed (in the worst case counteracted) by size effects.

It should be noted that particles from different classes may also have similar or identical magnetic susceptibilities but different sizes (which would use the differentiating effect of size without interference from susceptibility), or that both size and magnetic susceptibility may vary from class to class.

To achieve the binding of label particles to each other via a class-specific target component, the label particles may optionally be coated with class-specific binding agents. Typical examples for such (bioactive) agents are: antibodies, proteins, cells, DNA, RNA, small molecules, tissues, viruses.

BRIEF DESCRIPTION OF THE DRAWINGS

These and other aspects of the invention will be apparent from and elucidated with reference to the embodiment (s) described hereinafter. These embodiments will be described by way of example with the help of the accompanying drawings in which:

Figure 1 schematically shows an apparatus according to the present invention;
Figure 2 schematically illustrates different types of clusters that may play a role in a method according to the invention.

Like reference numbers in the Figures refer to identical or similar components.

DESCRIPTION OF PREFERRED EMBODIMENTS

Immunoassays exploiting (super)paramagnetic particles are important techniques to perform in vitro tests to detect particular compounds, due to the specificity of the antigen-antibody binding. These tests can be performed in various ways on a surface or in the liquid phase (bulk). Cluster assays are a class of assays in which the amount of formed particle clusters is indicative of the presence and/or concentration of biological components in the sample. Cluster assays are particularly attractive because of the formation of a biological binding in the bulk of the fluid without involving any interaction with a surface: they are easier to make, faster and also cheaper.

One type of cluster assay uses magnetic nanoparticles, which can be described as polymer spheres in which magnetic material in the form of nanometer-sized grains is embedded. A main advantage is that they can be actuated exploiting an external magnetic field. Hence it is possible to considerably speed up the formation of the biological binding, making the assay faster and improving the sensitivity.

In order to design a fast cluster assay, inter alia the issues of sensitivity and multiplexing must be addressed. The present invention is based on the assumption that the unique dynamic of rotation and/or oscillation of clusters formed by superparamagnetic beads, particularly of two-particle clusters, can solve these issues.

Regarding the lack of high sensitivity to detect biological reactions, that is the need of rising the limit of sensitivity of the biosensor, the aforementioned cluster dynamics can be exploited by an actuation scheme applying oscillating or (partially) rotating magnetic fields with time-variable amplitude. This is based on the finding that an oscillating/rotating magnetic field with a varying field amplitude can couple selectively to bound clusters. If the field characteristics are chosen properly, the field does not rotate larger clusters and it does not generate novel clusters during the actuation. In general, the complex rotational behavior involves three different regimes: initially, the superparamagnetic beads can rotate at the same frequency as the external field, but with a frequency-dependent phase-lag. Once the phase-lag reaches 90 degrees, the bead is experiencing the maximum torque available (at the so-called "breakdown frequency" or "critical frequency"). If the external frequency is increased further, the coupling between the external field and the superparamagnetic beads becomes more and more inefficient and the beads start to slow down. Furthermore, a wiggling rotation of the clusters can be observed: a backward oscillation superimposes the smooth rotation of the superparamagnetic beads. At even higher frequencies, the beads are able to rotate again due to the presence of nanometric grains of ferromagnetic material within the superparamagnetic beads.

In order to achieve effective multiplexing, i.e. to be able to detect different biological entities at once as fast as possible, a detectable parameter affecting the rotation (or, more generally, the actuation) of clusters formed through different biological reagents must be found. It is proposed here to detect differences in oscillation behavior (e.g. in angular velocities) of clusters formed by different biological entities when exposed to an external magnetic field at a fixed frequency. The detection can for instance be performed optically in transmission or refractive mode.

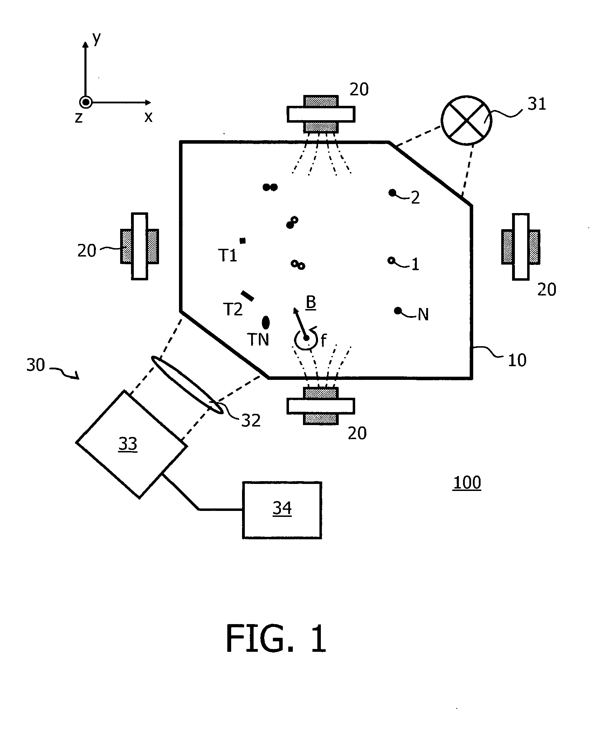

Figure 1 schematically shows an apparatus 100 with which the aforementioned approach can be realized. The apparatus 100 comprises the following main components:

1) A sample chamber 10 in which a sample with target components to be detected can be provided. In the shown example, N different target components T1, T2, ... TN are indicated, which may for example represent different antibodies, DNA-strands or the like. The sample chamber 10 will typically be a disposable unit that can for example be made from plastic by injection molding. To allow optical examinations, the walls of the sample chamber 10 are preferably transparent.

2) A magnetic field generator 20, here realized by a quadrupole with four magnetic coils 20 arranged at right angles to each other. As known to a person skilled in the art, supplying the coils 20 with electrical currents according to an appropriate schedule can generate an oscillating and/or rotating magnetic field B with time-varying field amplitude in the sample chamber 10. In the present example, the magnetic field will be assumed to be rotating and to have the following more specific form with a rotation frequency f:

$$\underline{B} = B_0 \cdot \begin{pmatrix} \sin(2\pi f \cdot t) \\ a \cdot \cos(2\pi f \cdot t) \end{pmatrix} \qquad (2)$$

3) An optical detection system 30 comprising, in this example, a light source 31 arranged at one side of the sample chamber 10 and focusing optics 32 arranged at the opposite side of the sample chamber for guiding light that was transmitted through the sample chamber onto a detector unit 33. The detector unit 33 may comprise any suitable sensor or plurality of sensors by which light of a given spectrum can be detected, for example photodiodes, photo resistors, photocells, a CCD or CMOS chip, or a photo multiplier tube. It provides a signal S indicative of the measured amount (e.g. intensity) of transmitted light, which is communicated to an evaluation unit 34 (e.g. a digital data processing unit) for further evaluation.

Superparamagnetic particles can be coated with biological entities (antibodies, DNA, cells, proteins, molecules) that selectively bind to other biological entities (analytes), for example the target components T1, T2, ... TN that shall be detected. A way to perform a cluster assay is then to bind the target components between two magnetic label particles, forming a "sandwich". The bond is strong enough to prevent the breaking of such two-particle clusters when they are actuated with an external rotating magnetic field. It should be noted that magnetic particles exposed to an external magnetic field also tend to form magnetically-induced clusters, but that this can be controlled/prevented using fields with a modulation of the amplitude in time.

The detection of different target components T1, T2, ... TN in one single measurement can now be achieved using different kinds of magnetic label particles in the same sample chamber where the detection is taking place. The particles preferably have the same dimensions, but a different magnetic content.

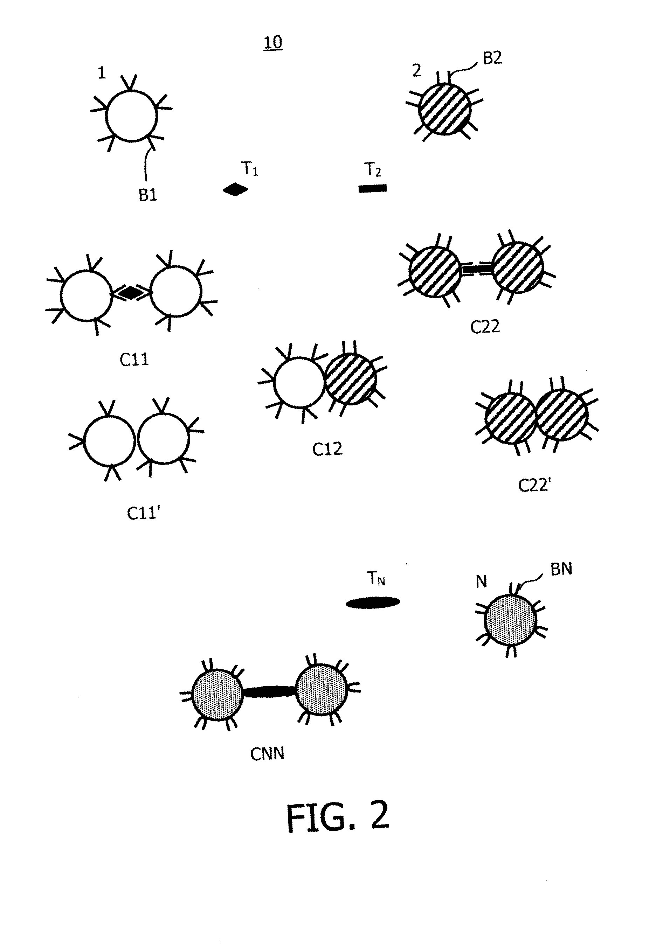

Figure 2 illustrates in this respect in more detail how the apparatus 100 can be used to distinguish between target specific clusters that are generated when an appropriate test kit of magnetic label particles is added to the sample that shall be investigated. In the discussed example, said test kit comprises two classes of magnetic label particles (superparamagnetic beads), wherein the magnetic label particles 1, 2, ... N within each class have class-specific binding sites B1, B2, ... BN for the different target components T1, T2, ... TN, respectively. Moreover, particles of the two classes shall have different magnetic susceptibilities $\chi_1$, $\chi_2$, ... $\chi_N$, respectively.

Due to their target specific binding sites B1, magnetic label particles 1 of the first class can form stable, specifically bound two-particle clusters C11 via an intermediate target component T1. Similarly, magnetic label particles 2 from the second class can form stable, specifically bound two-particle clusters C22 via an intermediate second target component T2, etc. Finally, magnetic label particles N from the N-th class can form stable, specifically bound two-particle clusters CNN via an intermediate N-th target component TN.

Figure 2 further illustrates two-particle clusters C 12, C11', and C22' in which two different magnetic label particles 1 and 2 or two magnetic label particles 1 or 2 of the same class are directly (unspecifically) bound without an intermediate target component.

The clusters $C_{11}$, $C_{22}$ etc. are clusters of two superparamagnetic particles with a magnetic susceptibility, i.e. torque and rotation in an external magnetic field B are generated by induced magnetic moments and a shape anisotropy of the cluster. The magnetic grains in a superparamagnetic particle gain an induced magnetic moment that provides the energy needed to rotate a clusters through the coupling with the external field. The rotational behavior of the clusters is characterized by the presence of a critical frequency $f_c$, beyond which the rotation is not synchronous with the external field anymore. Theoretical modeling of these processes yields the following predicted value of the critical frequency:

$$f_c = \frac{1}{2\pi} \cdot \frac{\frac{1}{6}\chi_1\chi_2 B^2}{28\eta\mu_0} \; ,$$

where $\eta$ is the viscosity of the fluid medium, $\mu_0 = 4\pi\cdot 10^{-7}$ H/m, B is the modulus of the applied magnetic field, and $\chi_i$ is the susceptibility of the i-th particle in the cluster.

In view of this background, it is suggested to exploit a number $N \geq 2$ of different magnetic label particles (preferably with the same dimension, but with different susceptibility $\chi$) to detect N different species of target components.

In the following, the easiest case of N = 2 will be considered in more detail. In this case three different types of clusters can be formed in the sample volume (assuming that $\chi_1 < \chi_2$):

"type 1": Clusters C 11 of two particles 1 with low susceptibility $\chi_1$;
"type 3": Clusters C22 of two particles 2 with high susceptibility $\chi_2$;
"type 2": Clusters C12 of one particle 1 with low susceptibility $\chi_1$ and one particle 2 with high susceptibility $\chi_2$. These clusters will only be magnetically coupled with no target component T1 or T2 in the "sandwich".

Each of these clusters is then characterized by a different critical frequency $f_{c1} < f_{c2} < t_{c3}$:

$$f_{c1} = \frac{1}{2\pi} \cdot \frac{\frac{1}{6}\chi_1^2 B^2}{28\eta\mu_0} \; , \quad f_{c2} = \frac{1}{2\pi} \cdot \frac{\frac{1}{6}\chi_1\chi_2 B^2}{28\eta\mu_0} \; , \quad f_{c3} = \frac{1}{2\pi} \cdot \frac{\frac{1}{6}\chi_2^2 B^2}{28\eta\mu_0} \; .$$

Once the clusters are formed, a way to detect them is to perform a sweep of the rotation frequency f of the applied rotating magnetic field for a given value of the magnetic field amplitude $B_0$ (cf. formula (2)). At low frequencies all the clusters will rotate synchronously with the external field. For frequencies above $f_{c1}$ the "type 1" clusters C11 are expected to be characterized by a lower angular velocity than the other clusters. Above $f_{c2}$ only the "type 3" clusters C22 will still be able to rotate synchronously with the field. In this way, detecting (e.g. optically) the number of clusters rotating below $f_{c1}$, one can obtain an esteem of the total number of clusters C11, C12, C22 of types 1, 2, 3 present in the sample chamber. Then detecting at a frequency f between $f_{c1}$ and $f_{c2}$ leads to the determination of the number of clusters C12 and C22 of the type 2 and 3, while detecting between $f_{c2}$ and $f_{c3}$ gives the number of clusters C22 of type 3. Clearly the number of clusters C11 or C22 is proportional to the concentration of the corresponding target component T1 or T2, respectively, in the sample.

This mechanism can be extended to an arbitrary large number N of different magnetic particles and target components, the limit being in practice only the actual possibility of finding (commercially available) particles with susceptibility different enough to give sensitive differences in the critical frequencies.

It is not necessary that all particles forming a cluster are magnetic, because it suffices for magnetic actuation that at least one particle of a cluster is magnetic. The prefered embodiment is however that as many particles of a cluster as possible are magnetic, because this facilitates the magnetic actuation.

Experiments using magnetic particles of I $\mu$m in diameter suspended in a saline solution of viscosity $\eta = 1\cdot 10^{-3}$ Pa·s and using a magnetic field with $B_0 = 1.58$ mT have proven the validity of the above concepts. The presence of the three different types of clusters could be observed, recognizable through different rotational behaviors when actuating at opportune frequencies, as described above. Type 2 clusters were additionally easily recognizable because of an asymmetric way of rotating, due to the fact that the "magnetic centre of mass" is not in the geometrical centre, but closer to the centre of the particle with higher susceptibility. Critical frequencies of $f_{c1}$ = 4 Hz, $f_{c2}$ = 6 Hz, and $f_{c3}$ = 14 Hz were determined.

The described innovative way to achieve multiplexing in cluster assays has still further advantages. During experiments it has been observed that the unspecific (magnetically induced) clusters formed by different particles ("type 2" clusters C12) are less stable than the unspecific clusters formed by particles of the same kind (clusters C11' and C22', which may be assigned to a "type 4"). Moreover, the only source of noise is given by the latter unspecific clusters that are formed by particles with the same susceptibility ("type 4") because they cannot be distinguished from the specific ones in terms of angular velocity. Actuating with a magnetic field with modulated amplitude will easily break "type 2" clusters, but will also reduce the presence of the "type 4" clusters, hence greatly improving the sensitivity. On top of this, the expected number of "type 4" clusters is lower than in the case of using a single kind of magnetic particles, because on average the total number of unspecific clusters formed can be considered constant, but now some of them are "type 2" clusters and can be broken or detected.

A particular aspect of the described method is to use one magnetic field component to create alignment of clusters, and another stronger magnetic field component to create repulsive forces. In other words, the concept is to create a preferred orientation for the clusters (i.e. along the weak component) and use the strong component to polarize the particles with moments orthogonal to the axis, creating a repulsive force. In general, a large variety of fields is able to create such a repulsive configuration:

- The weak component may for example be a constant field, a weak sinusoidal wave, or a square wave. The amplitude is preferably tuned so that only the smallest clusters can respond to the field.
- The stronger component may for example be a sinusoidal wave or a sequence of pulses. The amplitude is preferably tuned in such a way that it is not completely dominant (maximum ten times bigger than the amplitude of the weaker component) over the weaker component of the field.

The frequency of the two components can be different (i.e. square waves at 10 Hz can be combined with sinusoidal waves at 4 Hz). In the above actuations, the motion of the clusters is often oscillatory and they do not perform full rotations. The percentage of non-specifically bound clusters showing a breaking event observed in experiments is of the order of 20-30 %.

In conclusion, the overall magnetic field does not need to be fully rotating (as described in equation (2)), i.e. the method also works for non-sinusoidal field components and an overall field that is partially rotating. Moreover, the oscillation frequency of the overall field should be lower than about 10-times the inverse of the alignment time of the clusters for the given experimental parameters (field amplitude, viscosity, magnetic content of the particles etc.), which is related to the critical frequency.

While the invention was described above with reference to particular embodiments, various modifications and extensions are possible, for example:

- Molecular targets often determine the concentration and/or presence of larger moieties, e.g. cells, viruses, or fractions of cells or viruses, tissue extract, etc.
- Measurement data can be derived as an end-point measurement, as well as by recording signals kinetically or intermittently.
- The apparatus and method can be used as rapid, robust, and easy to use point-of-care biosensors for small sample volumes. The reaction chamber can be a disposable item to be used with a compact reader, containing the one or more field generating means and one or more detection means. Also, the apparatus and methods of the present invention can be used in automated high-throughput testing. In this case, the reaction chamber is e.g. a well-plate or cuvette, fitting into an automated instrument.
- With nano-particles are meant particles having at least one dimension ranging between 3 nm and 5000 nm, preferably between 10 nm and 3000 nm, more preferred between 50 nm and 1000 nm.

Finally, it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

**Claims**

1. A method for detecting a number of N $\geq$ 2 different species of target components (T1, T2, ... TN) in a sample, comprising:

a) adding N classes of label particles (1, 2, ...N) to the sample, wherein at least one of the label particles (1, 2, ...N) in each class is a magnetic particle, and wherein label particles from each class can bind to label particles from the same class via a class-specific species of target component (T1, T2, ... TN);
b) allowing the label particles (1, 2, ...N) to form clusters (C11, C22, C12, C11', C22', C12', ... CNN);
c) selectively actuating different types of said clusters to perform an oscillating motion or a fully rotating motion by generating a time-varying magnetic field (B);
d) detecting the selectively actuated clusters.

2. An apparatus (100) for detecting a number of N $\geq$ 2 different species of target components (T1, T2, ... TN) in a sample, comprising:

a) a sample chamber (10) for accommodating the sample;
b) a magnetic field generator (20) for applying a time-varying magnetic field (B) to the sample chamber, wherein the magnetic field is adapted to selectively actuate different types of clusters (C11, C22, C12, C11', C22', C12', ... CNN) to perform an oscillating motion or a fully rotating motion, wherein the clusters comprise at least one class of label particles (1, 2, ... N), wherein at least one of the label particles (1, 2, ...N) in each class is a magnetic particle, and wherein label particles from each class can bind to label particles from the same class via a class-specific species of target component (T1, T2, ... TN);
c) a detection system (30) for detecting selectively actuated clusters.

3. The method according to claim 1 or the apparatus (100) according to claim 2,
**characterized in that** said magnetic field (B) comprises at least one oscillating component, wherein the field amplitude varies over time.

4. The method according to claim 1 or the apparatus (100) according to claim 2,
**characterized in that** only clusters (C 11, C22, C12, C11', C22', C12', ... CNN) up to a predetermined size are actuated by said magnetic field (B).

5. The method according to claim 1 or the apparatus (100) according to claim 2,
**characterized in that** only clusters (C 11, C22, C12, C11', C22', C12', ... CNN) consisting of two label particles (1, 2, ... N) are actuated by said magnetic field (B).

6. The method according to claim 1 or the apparatus (100) according to claim 2,
**characterized in that** the ratio between the maximum and minimum amplitudes of the magnetic field (B) is between 1.1 and 10, preferably between 2 and 8, and most preferably between 4 and 6.

7. The method or the apparatus (100) according to claim 3,
**characterized in that** the detection of selectively actuated clusters (C11, C22, C12, C11', C22', C12', ... CNN) comprises the detection of an oscillation and/or a rotation synchronously to the oscillating component of the magnetic field (B).

8. The method according to claim 1 or the apparatus (100) according to claim 2,
**characterized in that** the magnetic field is a rotating magnetic field (B), wherein the rotational frequency (f) can preferably be swept over a given range.

9. The method or the apparatus (100) according to claim 8,
**characterized in that** said range comprises at least one frequency above and one frequency below a critical frequency at which one type of clusters (C11, C22, C12, C11', C22', C12', ... CNN) changes its reaction to the rotating magnetic field (B).

10. The method according to claim 1 or the apparatus (100) according to claim 2,
**characterized in that** selectively actuated clusters (C 11, C22, C12, C 11', C22', C12', ... CNN) are optically detected.

11. The method according to claim 1 or the apparatus (100) according to claim 2,
**characterized in that** the actuation of the clusters (C11, C22, C12, C11', C22', C12', ... CNN) comprises the breaking of non-specifically bound clusters (C12, C11', C22', C12').

**12.** The method according to claim 1 or the apparatus (100) according to claim 2,
**characterized in that** all particles of at least one class are magnetic particles (1, 2, ... N).

**13.** The method according to claim 1 or the apparatus (100) according to claim 2,
**characterized in that** label particles (1, 2, ... N) from different classes have substantially the same size.

**14.** The method according to claim 1 or the apparatus (100) according to claim 2,
**characterized in that** the label particles (1, 2, ... N) are coated with a class-specific binding agent for target particles
(T1, T2, ... TN).

**Patentansprüche**

**1.** Verfahren zum Nachweis einer Anzahl von N ≥2 verschiedenen Arten von Zielkomponenten (T1, T2, ... TN) in einer
Probe, das Folgendes umfasst:

a) Hinzufügen von N Klassen mit Markierungsteilchen (1, 2, ... N) zu der Probe, wobei zumindest eines der
Markierungsteilchen (1, 2, ... N) in jeder Klasse ein Magnetteilchen ist und wobei sich Markierungsteilchen aus
jeder Klasse über eine klassenspezifische Art von Zielkomponente (T1, T2, ... TN) an Markierungsteilchen
derselben Klasse binden können,
b) Zulassen, dass die Markierungsteilchen (1, 2, ... N) Cluster (C11, C22, C12, C11', C22', C12', ... CNN) bilden,
c) selektives Erregen verschiedener Arten der genannten Cluster durch die Erzeugung eines zeitlich veränderlichen Magnetfeldes (B), so dass sie eine oszillierende Bewegung oder eine vollständig rotierende Bewegung
ausführen,
d) Nachweisen der selektiv erregten Cluster.

**2.** Vorrichtung (100) zum Nachweis einer Anzahl von N ≥ 2 verschiedenen Arten von Zielkomponenten (T1, T2, ... TN)
in einer Probe, wobei die Vorrichtung Folgendes umfasst:

a) eine Probenkammer (10) zur Aufnahme der Probe,
b) einen Magnetfelderzeuger (20) zum Anlegen eines zeitlich veränderlichen Magnetfeldes (B) an die Probenkammer, wobei das Magnetfeld so ausgelegt ist, dass es selektiv verschiedene Arten von Clustern (C 11, C22,
C12, C11', C22', C12', ... CNN) erregt, so dass sie eine oszillierende Bewegung oder eine vollständig rotierende
Bewegung ausführen, wobei die Cluster zumindest eine Klasse mit Markierungsteilchen (1, 2, ... N) enthalten,
wobei zumindest eines der Markierungsteilchen (1, 2, ... N) in jeder Klasse ein Magnetteilchen ist und wobei
sich Markierungsteilchen aus jeder Klasse über eine klassenspezifische Art von Zielkomponente (T1, T2, ...
TN) an Markierungsteilchen derselben Klasse binden können,
c) Nachweissystem (30) für den Nachweis von selektiv erregten Clustern.

**3.** Verfahren nach Anspruch 1 oder Vorrichtung (100) nach Anspruch 2, **dadurch gekennzeichnet, dass** das genannte
Magnetfeld (B) zumindest eine oszillierende Komponente enthält, wobei sich die Feldamplitude über die Zeit verändert.

**4.** Verfahren nach Anspruch 1 oder Vorrichtung (100) nach Anspruch 2, **dadurch gekennzeichnet, dass** nur Cluster
(C11, C22, C12, C11', C22', C12', ... CNN) bis zu einer vorbestimmten Größe von dem genannten Magnetfeld (B)
erregt werden.

**5.** Verfahren nach Anspruch 1 oder Vorrichtung (100) nach Anspruch 2, **dadurch gekennzeichnet, dass** nur Cluster
(C11, C22, C12, C11', C22', C12', ... CNN), die aus zwei Markierungsteilchen (1, 2, ... N) bestehen, von dem
genannten Magnetfeld (B) erregt werden.

**6.** Verfahren nach Anspruch 1 oder Vorrichtung (100) nach Anspruch 2, **dadurch gekennzeichnet, dass** das Verhältnis
zwischen der maximalen und der minimalen Amplitude des Magnetfeldes (B) zwischen 1,1 und 10, vorzugsweise
zwischen 2 und 8 und am meisten bevorzugt zwischen 4 und 6 beträgt.

**7.** Verfahren nach Anspruch 1 oder Vorrichtung (100) nach Anspruch 3, **dadurch gekennzeichnet, dass** der Nachweis
der selektiv erregten Cluster (C 11, C22, C12, C11', C22', C12', ... CNN) den Nachweis einer mit der oszillierenden
Komponente des Magnetfeldes (B) synchronen Oszillation und/oder Rotation umfasst.

**8.** Verfahren nach Anspruch 1 oder Vorrichtung (100) nach Anspruch 2, **<u>dadurch gekennzeichnet, dass</u>** das Magnetfeld ein rotierendes Magnetfeld (B) ist, wobei die Drehfrequenz (f) vorzugsweise in einem gegebenen Bereich verändert werden kann.

**9.** Verfahren nach Anspruch 1 oder Vorrichtung (100) nach Anspruch 8, **<u>dadurch gekennzeichnet, dass</u>** der genannte Bereich zumindest eine Frequenz über und eine Frequenz unter einer kritischen Frequenz umfasst, bei der eine Art von Clustern (C11, C22, C12, C11', C22', C12', ... CNN) seine Reaktion auf das rotierende Magnetfeld (B) ändert.

**10.** Verfahren nach Anspruch 1 oder Vorrichtung (100) nach Anspruch 2, **<u>dadurch gekennzeichnet, dass</u>** die selektiv erregten Cluster (C 11, C22, C12, C11', C22', C12', ... CNN) optisch nachgewiesen werden.

**11.** Verfahren nach Anspruch 1 oder Vorrichtung (100) nach Anspruch 2, **<u>dadurch gekennzeichnet, dass</u>** die Erregung der Cluster (C 11, C22, C12, C11', C22', C12', ... CNN) das Aufbrechen von nicht spezifischen gebundenen Clustern (C12, C11', C22', C12') umfasst.

**12.** Verfahren nach Anspruch 1 oder Vorrichtung (100) nach Anspruch 2, **<u>dadurch gekennzeichnet, dass</u>** alle Teilchen zumindest einer Klasse Magnetteilchen (1, 2, ... N) sind.

**13.** Verfahren nach Anspruch 1 oder Vorrichtung (100) nach Anspruch 2, **<u>dadurch gekennzeichnet, dass</u>** die Markierungsteilchen (1, 2, ... N) aus verschiedenen Klassen im Wesentlichen die gleiche Größe haben.

**14.** Verfahren nach Anspruch 1 oder Vorrichtung (100) nach Anspruch 2, **<u>dadurch gekennzeichnet, dass</u>** die Markierungsteilchen (1, 2, ... N) mit einem klassenspezifischen Bindemittel für Zielteilchen (T1, T2, ... TN) überzogen sind.

**Revendications**

**1.** Procédé pour détecter un nombre de $N \geq 2$ espèces différentes de composants cibles (T1, T2, ... TN) dans un échantillon, comprenant :

a) ajouter N classes de particules de marquage (1, 2, ...N) à l'échantillon, dans lequel au moins une des particules de marquage (1, 2, ...N) dans chaque classe est une particule magnétique, et dans lequel des particules de marquage de chaque classe peuvent se lier à des particules de marquage de la même classe par l'intermédiaire d'une espèce de classe spécifique de composant cible (T1, T2, ... TN) ;
b) permettre aux particules de marquage (1, 2, ...N) de former des agrégats (C11, C22, C12, C11', C22', C12', ... CNN);
c) actionner sélectivement différents types desdits agrégats pour réaliser un mouvement oscillant ou un mouvement complètement rotatif en générant un champ magnétique variant en temps (B) ;
d) détecter les agrégats sélectivement actionnés.

**2.** Appareil (100) pour détecter un nombre de $N \geq 2$ espèces différentes de composants cibles (T1, T2, ... TN) dans un échantillon, comprenant :

a) une chambre à échantillon (10) pour loger l'échantillon ;
b) un générateur de champ magnétique (20) pour appliquer un champ magnétique variant en temps (B) sur la chambre à échantillon, dans lequel le champ magnétique est adapté pour actionner sélectivement différents types d'agrégats (C11, C22, C12, C11', C22', C12', ... CNN) pour réaliser un mouvement oscillant ou un mouvement complètement rotatif, dans lequel les agrégats comprennent au moins une classe de particules de marquage (1, 2, ... N), dans lequel au moins une des particules de marquage (1, 2, ...N) dans chaque classe est une particule magnétique, et dans lequel des particules de marquage de chaque classe peuvent se lier à des particules de marquage de la même classe par l'intermédiaire d'une espèce de classe spécifique de composant cible (T1, T2, ... TN) ;
c) un système de détection (30) pour détecter sélectivement des agrégats actionnés.

**3.** Procédé selon la revendication 1 ou appareil (100) selon la revendication 2, **caractérisé en ce que** ledit champ magnétique (B) comprend au moins un composant oscillant, dans lequel l'amplitude de champ varie avec le temps.

**4.** Procédé selon la revendication 1 ou appareil (100) selon la revendication 2, **caractérisé en ce que** seulement des agrégats (C11, C22, C 12, C11', C22', C12', ... CNN) jusqu'à une taille prédéterminée sont actionnés par ledit champ magnétique (B).

5. Procédé selon la revendication 1 ou appareil (100) selon la revendication 2, **caractérisé en ce que** seulement des agrégats (C11, C22, C 12, C11', C22', C12', ... CNN) constitués de deux particules de marquage (1, 2, ... N) sont actionnés par ledit champ magnétique (B).

6. Procédé selon la revendication 1 ou appareil (100) selon la revendication 2, **caractérisé en ce que** le rapport entre les amplitudes maximum et minimum du champ magnétique (B) est entre 1,1 et 10, mieux encore entre 2 et 8, et idéalement entre 4 et 6.

7. Procédé ou appareil (100) selon la revendication 3, **caractérisé en ce que** la détection d'agrégats actionnés sélectivement (C11, C22, C12, C11', C22', C12', ... CNN) comprend la détection d'une oscillation et/ou d'une rotation de façon synchrone à la composante oscillante du champ magnétique (B).

8. Procédé selon la revendication 1 ou appareil (100) selon la revendication 2, **caractérisé en ce que** le champ magnétique est un champ magnétique rotatif (B), dans lequel la fréquence de rotation (1) peut de préférence être balayée sur une plage donnée.

9. Procédé ou appareil (100) selon la revendication 8, **caractérisé en ce que** ladite plage comprend au moins une fréquence au-dessus et une fréquence en-dessous d'une fréquence critique à laquelle un type d'agrégats (C11, C22, C12, C11', C22', C12', ... CNN) change sa réaction au champ magnétique rotatif (B).

10. Procédé selon la revendication 1 ou appareil (100) selon la revendication 2, **caractérisé en ce que** des agrégats actionnés sélectivement (C11, C22, C12, C11', C22', C12', ... CNN) sont détectés optiquement.

11. Procédé selon la revendication 1 ou appareil (100) selon la revendication 2, **caractérisé en ce que** l'actionnement des agrégats (C11, C22, C12, C11', C22', C12', ... CNN) comprend la rupture d'agrégats liés non spécifiquement (C12, C11', C22', C12').

12. Procédé selon la revendication 1 ou appareil (100) selon la revendication 2, **caractérisé en ce que** toutes les particules d'au moins une classe sont des particules magnétiques (1, 2, ... N).

13. Procédé selon la revendication 1 ou appareil (100) selon la revendication 2, **caractérisé en ce que** les particules de marquage (1, 2, ... N) de classes différentes possèdent sensiblement la même taille.

14. Procédé selon la revendication 1 ou appareil (100) selon la revendication 2, **caractérisé en ce que** les particules de marquage (1, 2, ... N) sont enduites avec un agent de liage de classe spécifique pour des particules cibles (T1, T2, ... TN).

FIG. 1

## FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080206104 A1
- US 2006040286 A1
- US 5932097 A
- WO 2009091643 A1
- WO 2009037636 A1
- EP 08105253 A